# FASCICULE DE BREVET EUROPEEN

(11) **EP 2 898 907 B1**
(45) Date de publication et mention de la délivrance du brevet: **02.03.2022**
(21) Numéro de dépôt: 14196953.5
(22) Date de dépôt: 09.12.2014
(51) Int. Cl.: A61M 1/00

(54) **Dispositif régulateur de vide**
Vakuum-Einstellvorrichtung
Vacuum regulator device

(30) Priorité: 24.01.2014 FR 1450609
(43) Date de publication de la demande: 29.07.2015
(73) Titulaire: Air Liquide Medical Systems, 92160 Antony (FR)
(72) Inventeur: Rezel, Christophe, Roland, 91600 Savigny sur Orge (FR); Dutheil, Benjamin, 75001 Paris (FR); Harant, Catherine, 78370 Plaisir (FR)
(74) Mandataire: Air Liquide

(56) Documents cités:
- WO-A1-2012/161723
- US-A1- 2004 051 746
- US-B1- 6 364 853

## Description

La présente invention concerne un dispositif régulateur de vide.

L'invention concerne plus particulièrement un dispositif régulateur de vide comprenant un boîtier abritant un circuit de fluide s'étendant entre une entrée et une sortie, le circuit comprenant un organe de réglage du niveau de vide actionnable par un organe de sélection monté mobile sur le boîtier, le dispositif comprenant en outre un interrupteur pour commander la marche ou l'arrêt du dispositif via un clapet d'isolement situé sur le circuit, l'interrupteur comprenant un bouton poussoir actionnable manuellement, le bouton poussoir étant monté mobile sur le corps selon deux directions opposées entre deux positions correspondant respectivement aux positions de marche et d'arrêt, une première extrémité du bouton poussoir faisant saillie par rapport au boîtier en position d'arrêt tandis qu'une seconde extrémité du bouton poussoir fait saillie par rapport au boîtier en position de marche, le dispositif comprenant en outre un indicateur visuel de la position de marche ou d'arrêt du bouton poussoir.

L'invention concerne l'affichage du fonctionnement marche/arrêt (« ON/OFF ») d'un régulateur de vide.

Les documents FR2880434A1 et FR2880281A1 décrivent des exemples de régulateurs de vide. Le document US 6364853 B1 décrire d'exemple d'un dispositif commutable pour la régulation de l'irrigation d'un flux fluide ainsi que pour la régulation d'vide.

Les régulateurs de vide comprennent généralement un vacuomètre qui sert à régler une dépression en faisant fonctionner l'appareil. Ceci permet également de manipuler le dispositif et de vérifier que le régulateur de vide est capable d'aspirer pour être sûr de son fonctionnement.

Les régulateurs de vide connus, et notamment celui commercialisé par la demanderesse sous la marque Lagoon^{®}, présentent des couleurs sur le bouton poussoir signalant la position de « marche ou d'« arrêt ».

Par exemple, l'opérateur doit appuyer sur le bouton vert pour la mise en marche (en dépression) et l'autre extrémité du bouton (rouge) apparaît tandis que le bouton vert s'escamote dans le boîtier. La partie rouge correspond à la prochaine action (mise à l'arrêt en appuyant sur le bouton rouge pour faire apparaître à nouveau l'extrémité verte).

Un problème posé par cet agencement est que la couleur rouge apparente peut penser à l'utilisateur que le dispositif est à l'arrêt alors qu'il est en marche. D'autres dispositifs régulateurs de vide utilisent un code couleur inversé ou pas de code couleur.

Toutes ces solutions connues ne permettent ainsi pas à l'utilisateur d'identifier rapidement et de manière non équivoque si le régulateur de vide est en fonctionnement ou à l'arrêt.

Un but de la présente invention est de pallier tout ou partie des inconvénients de l'art antérieur relevés ci-dessus.

A cette fin, le dispositif selon l'invention, par ailleurs conforme à la définition générique qu'en donne le préambule ci-dessus, est essentiellement caractérisé en ce que l'indicateur visuel de position comprend support d'informations distinct des première et seconde extrémités du bouton poussoir, le support d'information actionné par le déplacement avec bouton poussoir et mettant en évidence respectivement une première information ou une seconde information selon que le bouton poussoir est en position de marche ou en position d'arrêt.

Selon cet agencement, le dispositif comporte un affichage ergonomique et fiable du mode de fonctionnement (en marche/en arrêt) du dispositif.

Par ailleurs, des modes de réalisation de l'invention peuvent comporter l'une ou plusieurs des caractéristiques suivantes :
- le support d'information est solidaire en déplacement avec bouton poussoir,
- le boîtier comporte une fenêtre située sur le trajet du support d'information lors de ses déplacements avec bouton poussoir et en ce que seule la première information ou la seconde information du support d'information coïncide avec la fenêtre selon respectivement que le bouton poussoir est en position de marche ou en position d'arrêt,
- le bouton poussoir est mobile en translation au travers du boîtier selon un axe de translation, les deux extrémités du bouton poussoir débouchant respectivement sur deux faces latérales opposées du boîtier, la première extrémité du bouton poussoir faisant saillie par rapport au boîtier lorsque la seconde extrémité du bouton poussoir est en retrait ou affleure par rapport au boîtier et inversement,
- le support d'informations comprend un panneau relié rigidement au reste du bouton poussoir,
- le support d'informations est décalé par rapport aux première et seconde extrémités du bouton poussoir selon une direction transversale à l'axe de translation,
- le support d'information met en évidence la première information ou la seconde information sur une face dite face avant du boîtier comportant l'organe de sélection,
- la première information et la seconde information sont distinctes et comprennent chacune au moins l'un parmi: au moins une couleur, un symbole alphanumérique, un texte,
- l'entrée et/ou la sortie du circuit comprend un raccord monté de façon démontable par rapport au boîtier, le boîtier comprenant un organe d'accrochage amovible d'un raccord pour maintenir ce dernier lorsqu'il n'est pas monté au niveau de l'entrée ou de la sortie,
- l'organe de sélection est situé sur une face dite avant du boîtier, le boîtier comportant une face arrière opposée à la face avant et en ce que ledit organe d'accrochage du raccord est situé sur une face du boîtier distincte de la face arrière,
- l'organe d'accrochage est situé sur face latérale du boîtier située entre les faces avant et arrière,
- l'organe d'accrochage comprend un mécanisme d'attache à déformation élastique faisant saillie sur la surface extérieure du boîtier,
- l'organe d'accrochage comprend un mécanisme d'attache à déformation élastique logé dans une cavité du boîtier destinée à accueillir au moins partiellement le raccord de sorte que le raccord est au moins partiellement logé dans le boîtier sans faire saillie sur la surface extérieure du boîtier,
- le dispositif comporte un vacuomètre affichant la valeur de vide régulé par le dispositif,
- l'organe d'accrochage comprend deux bras espacés et élastiquement déformables destinés à enserrer transversalement le raccord,
- l'organe d'accrochage comprend deux pions espacés dont l'un au moins est élastiquement déformable et destinés à enserrer le raccord au niveau de deux de ses extrémités opposées,
- le clapet d'isolement comprend un axe solidaire du bouton poussoir et coopérant avec le circuit,
- l'organe de réglage du niveau de vide actionnable par un organe de sélection comprend un clapet à ouverture progressive,

L'invention peut concerner également tout dispositif ou procédé alternatif comprenant toute combinaison des caractéristiques ci-dessus ou ci-dessous.

D'autres particularités et avantages apparaîtront à la lecture de la description ci-après, faite en référence aux figures dans lesquelles :
- la figure 1 représente une vue de face, schématique et partielle, d'un exemple de réalisation possible du dispositif selon l'invention dans un état d'arrêt,
- la figure 2 représente une vue similaire à la figure 1 dans laquelle le dispositif est en état de marche,
- la figure 3 représente en vue de côté, schématique et partielle d'un dispositif selon de la figure 1,
- la figure 4 représente une vue de face, schématique et partielle, illustrant un détail du dispositif des figures 1 à 3 illustrant un exemple de réalisation d'une partie du mécanisme de l'interrupteur du dispositif,
- les figures 5 et 6 représentent des vues de face, schématiques et partielles, illustrant un autre exemple de réalisation d'un dispositif selon l'invention muni d'un premier exemple de système d'attache de raccord,
- la figure 7 représente une vue de côté, schématique et partielle, illustrant un détail d'un autre exemple de réalisation d'un dispositif selon l'invention muni d'un second exemple de système d'attache de raccord dans lequel aucun raccord n'est accroché,
- la figure 8 représente une vue de face en transparence, schématique et partielle, d'un détail du dispositif de la figure 7 illustrant le second exemple de système d'attache de raccord dans lequel un raccord est accroché.

Le dispositif 1 régulateur de vide illustré à titre d'exemple non limitatif aux figures comprend un boîtier 2 abritant un circuit 3 de fluide s'étendant entre une entrée 4 de fluide et une sortie 5 de fluide. L'entrée 4, située par exemple une face 14 dite arrière du boîtier 2 est destinée à être raccordée par exemple avec une prise murale d'un circuit de vide d'un hôpital par exemple.

Le circuit 3 comprend un organe 6 de réglage du niveau de vide tel qu'un clapet à ouverture progressive. Cet organe 6 de réglage est actionnable par un organe 7 de sélection monté mobile sur le boîtier 2. L'organe 7 de sélection est par exemple un volant pivotant et est par exemple monté sur une face 13 dite avant du boîtier 2. La face avant 13 du boîtier 3 peut comporter également un vacuomètre 10, c'est-à-dire un indicateur du niveau de vide régulé par le dispositif (selon son état de marche/arrêt et la position de l'organe 7 de sélection).

Classiquement, le dispositif comprend en outre un interrupteur pour commander la marche ou l'arrêt du dispositif par exemple via un clapet 15 d'isolement situé sur le circuit 3. Le clapet 15 d'isolement comprend par exemple un axe ou « tiroir » solidaire du bouton 8 poussoir et coopérant avec le circuit 3.

L'interrupteur comprend un bouton 8 poussoir actionnable manuellement. Le bouton 8 poussoir est monté mobile sur le corps 2 selon deux directions opposées entre deux positions de préférence stables correspondant respectivement aux positions de marche et d'arrêt.

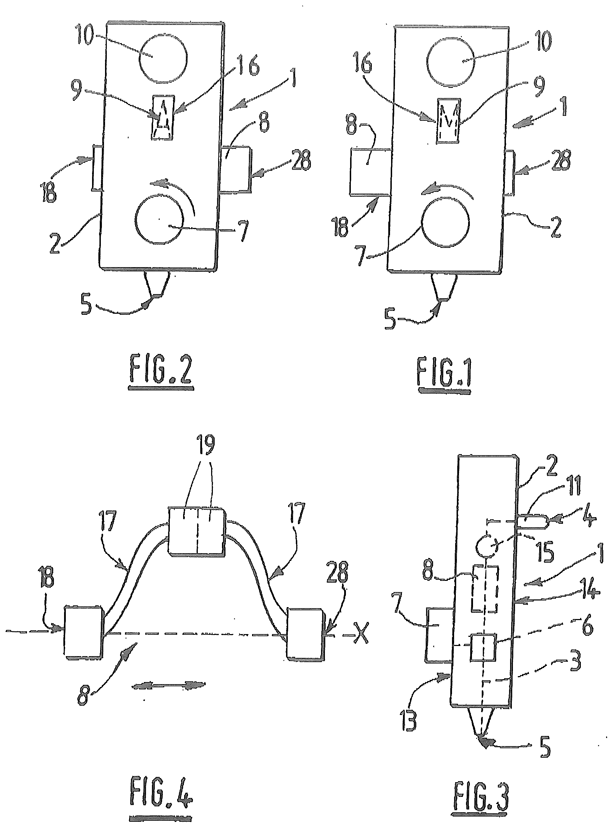

Dans la position de marche (M), une première extrémité 18 du bouton 8 poussoir fait saillie par rapport au boîtier 2 (cf. figure 1) tandis qu'une seconde extrémité 28 du bouton 8 poussoir est en retrait ou affleure ou fait plus faiblement saillie par rapport au boîtier 2. Inversement, dans la position d'arrêt (A), la première extrémité 18 du bouton 8 poussoir ne fait pas saillie ou faiblement saillie par rapport au boîtier 2 tandis que la seconde extrémité 28 du bouton 8 poussoir fait saillie par rapport au boîtier 2 (cf. figure 1).

Le dispositif comporte un indicateur 9 visuel de la position de marche ou d'arrêt du bouton 8 poussoir. Selon une caractéristique avantageuse, l'indicateur 9 visuel de position comprend support 19 d'informations distinct des première et seconde extrémités 18, 28 du bouton 8 poussoir. Ce support 9 d'information est solidaire en déplacement avec bouton 8 poussoir. Le support 9 d'information met en évidence sur le boîtier 2 respectivement une première information « M » ou une seconde information « A » selon que le bouton 8 poussoir est en position de marche ou en position d'arrêt.

Par exemple, le boîtier 2 comporte une fenêtre 16 (ouverture ou autre) située sur le trajet du support 9 d'information lors de ses déplacements avec bouton 8 poussoir. De préférence, seule la première information M ou la seconde information A du support 9 d'information coïncide avec la fenêtre (et est donc visible) selon respectivement que le bouton 8 poussoir est en position de marche ou en position d'arrêt.

La première information M et la seconde information A sont distinctes et comprennent par exemple chacune au moins l'un parmi: au moins une couleur (rouge/vert), un symbole alphanumérique (« Marche/Arrêt » ou « On/Off » ou « 0/1 ») ou tout autre représentation graphique appropriée.

Par exemple, le bouton 8 poussoir est mobile en translation au travers du boîtier 2 selon un axe X de translation perpendiculaire à l'axe vertical du dispositif. Les deux extrémités 18, 28 du bouton 8 poussoir débouchent de préférence respectivement sur deux faces latérales opposées du boîtier 2. En vue de face, la première extrémité 18 du bouton 8 poussoir fait saillie par rapport au boîtier 2 lorsque la seconde extrémité 28 du bouton 8 poussoir est en retrait ou affleure par rapport au boîtier 2 et inversement.

Le support 19 d'informations comprend par exemple un panneau relié rigidement au reste du bouton 8 poussoir, par exemple via des bras 17 de liaison rigides reliés respectivement aux deux extrémités 18, 28 (cf. figure 4).

Le support 19 d'informations est par exemple décalé par rapport aux première 18 et seconde 28 extrémités du bouton 8 poussoir selon une direction transversale à l'axe X de translation passant par les deux extrémités 18, 28.

De cette façon, la position du bouton 8 poussoir est indiquée de façon explicite sur la face avant du dispositif 1, sans nécessiter d'examiner la position du bouton 8 poussoir.

Dans une autre variante possible le support (19) d'informations distinct des première et seconde extrémités du bouton (8) poussoir peut être activé électriquement (par exemple via un système de capteur(s) de position) et peut comprendre un affichage électronique.

Comme visible à la figure 3, l'entrée 4 et/ou la sortie 5 du circuit 3 peut comprendre un raccord 11 (mâle) destiné à être raccordé à un appareillage et/ou un circuit. Le ou les raccords 11 peuvent être démontables.

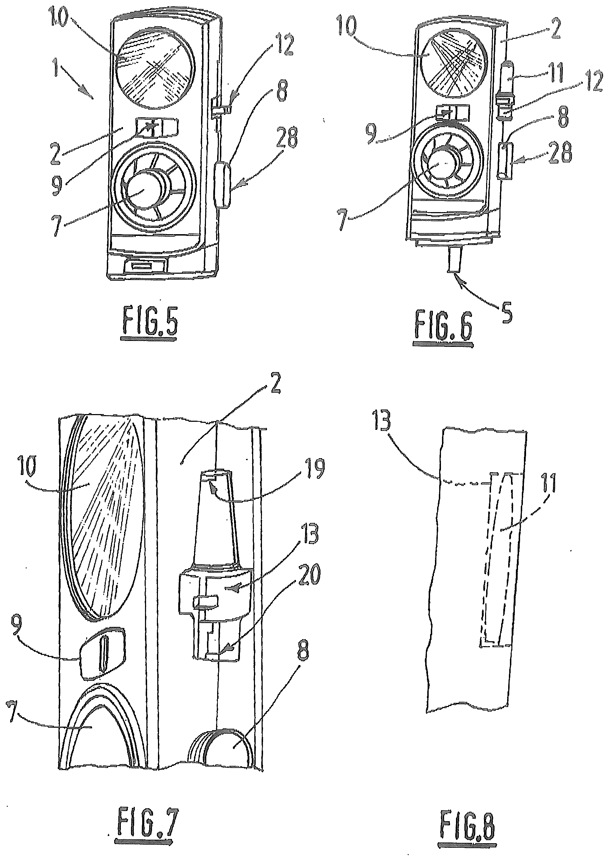

Comme illustré aux figures 5 à 8, le boîtier peut comprendre un organe 12, 13 d'accrochage amovible d'un raccord 11 pour maintenir ce dernier lorsqu'il n'est pas monté au niveau de l'entrée 4 ou de la sortie 5.

L'organe 12, 13 d'accrochage est situé de préférence sur face latérale du boîtier 2 (c'est-à-dire une face située entre les faces avant 13 et arrière 14). De cette façon, l'utilisateur voit immédiatement si le raccord est disponible (figure 5) ou absent de son support 12.

De préférence, l'organe 12 d'accrochage est adjacent à une extrémité 28 du bouton 8 poussoir.

L'organe 12 d'accrochage comprend par exemple un mécanisme d'attache à déformation élastique faisant saillie sur la surface extérieure du boîtier cf. figure 5. Cet organe 12 d'accrochage comprend par exemple deux bras ou crochets espacés et élastiquement déformable destinés à enserrer transversalement le raccord 11 (cf. figure 6).

En variante, et comme illustré aux figures 7 et 8, l'organe d'accrochage peut comprendre un mécanisme d'attache à déformation élastique logé dans une cavité 13 du boîtier 2 destinée à accueillir au moins partiellement le raccord 11. Ainsi, le raccord 11 peut faire partiellement saillie ou être complètement à l'intérieur du volume du boîtier 2 (cf. figure 8).

L'organe d'accrochage dans la cavité 13 peut comprendre par exemple deux pions 19, 20 ou languettes espacés dont l'un au moins est élastiquement déformable et destinés à enserrer le raccord 11 au niveau de deux de ses extrémités longitudinales opposées (par exemple à la manière de la fixation d'une pile cylindrique dans son logement).

La caractéristique (facultative) relative à l'organe 12, 13 d'accrochage du raccord peut être indépendante de la caractéristique ci-dessus relative au support 19 d'information. Ceci permet de retirer et maintenir visible un raccord d'une extrémité du dispositif lorsque ce raccord n'est pas utilisé.

Dans une variante de réalisation possible le bouton poussoir peut être remplacé par tout autre type de bouton, par exemple un bouton rotatif.

## Revendications

1. Dispositif régulateur de vide comprenant un boîtier (2) abritant un circuit (3) de fluide s'étendant entre une entrée (4) et une sortie (5), le circuit (3) comprenant un organe (6) de réglage du niveau de vide actionnable par un organe (7) de sélection monté mobile sur le boîtier (2), le dispositif comprenant en outre un interrupteur (8, 15) pour commander la marche ou l'arrêt du dispositif via un clapet (15) d'isolement situé sur le circuit (3), l'interrupteur comprenant un bouton (8) poussoir actionnable manuellement, le bouton (8) poussoir étant monté mobile sur le corps (2) selon deux directions opposées entre deux positions correspondant respectivement aux positions de marche et d'arrêt, **caractérisé en ce qu'**une première extrémité (18) du bouton (8) poussoir fait saillie par rapport au boîtier (2) en position d'arrêt tandis qu'une seconde extrémité (28) du bouton (8) poussoir fait saillie par rapport au boîtier (2) en position de marche, le dispositif comprenant en outre un indicateur (9) visuel de la position de marche ou d'arrêt du bouton (8) poussoir et **en ce que** l'indicateur (9) visuel de position comprend un support (19) d'informations distinct des première (18) et seconde extrémités (28) du bouton (8) poussoir, le support (9) d'informations étant actionné par le déplacement avec bouton (8) poussoir et mettant en évidence respectivement une première information (M) ou une seconde information (A) selon que le bouton (8) poussoir est en position de marche ou en position d'arrêt.

2. Dispositif selon la revendication 1, **caractérisé en ce que** le support (9) d'information est solidaire en déplacement avec bouton (8) poussoir.

3. Dispositif selon la revendication 1 ou 2, **caractérisé en ce que** le boîtier (2) comporte une fenêtre (16) située sur le trajet du support (9) d'information lors de ses déplacements avec bouton (8) poussoir et **en ce que** seule la première information (M) ou la seconde information (A) du support (9) d'information coïncide avec la fenêtre selon respectivement que le bouton (8) poussoir est en position de marche ou en position d'arrêt.

4. Dispositif selon l'une quelconque des revendications 1 à 3, **caractérisé en ce que** le bouton (8) poussoir est mobile en translation au travers du boîtier (2) selon un axe (X) de translation, les deux extrémités du bouton (8) poussoir débouchant respectivement sur deux faces latérales opposées du boîtier (2), la première extrémité (18) du bouton (8) poussoir faisant saillie par rapport au boîtier (2) lorsque la seconde extrémité (28) du bouton (8) poussoir est en retrait ou affleure par rapport au boîtier (2) et inversement.

5. Dispositif selon l'une quelconque des revendications 1 à 4, **caractérisé en ce que** le support (19) d'informations comprend un panneau relié rigidement au reste du bouton (8) poussoir.

6. Dispositif selon les revendications 4 et 5 prises en combinaison, **caractérisé en ce que** le support (19) d'informations est décalé par rapport aux première (18) et seconde (28) extrémités du bouton (8) poussoir selon une direction transversale à l'axe (X) de translation.

7. Dispositif selon l'une quelconque des revendications 1 à 6, **caractérisé en ce que** le support (9) d'information met en évidence la première information (M) ou la seconde information (A) sur une face dite face avant du boîtier (2) comportant l'organe (7) de sélection.

8. Dispositif selon l'une quelconque des revendications 1 à 7, **caractérisé en ce que** la première information (M) et la seconde information (A) sont distinctes et comprennent chacune au moins l'un parmi: au moins une couleur, un symbole alphanumérique, un texte.

9. Dispositif selon l'une quelconque des revendications 1 à 8, **caractérisé en ce que** l'entrée (4) et/ou la sortie (5) du circuit (3) comprend un raccord (11) monté de façon démontable par rapport au boîtier (2), le boîtier comprenant un organe (12, 13) d'accrochage amovible d'un raccord (11) pour maintenir ce dernier lorsqu'il n'est pas monté au niveau de l'entrée (4) ou de la sortie (5).

10. Dispositif selon la revendication 9, **caractérisé en ce que** l'organe (7) de sélection est situé sur une face dite avant (13) du boîtier (2), le boîtier (2) comportant une face (14) arrière opposée à la face (13) avant et **en ce que** ledit organe (12, 13) d'accrochage du raccord est situé sur une face du boîtier (2) distincte de la face (14) arrière.

11. Dispositif selon la revendication 10, **caractérisé en ce que** l'organe (12, 13) d'accrochage est situé sur face latérale du boîtier (2) située entre les faces avant (13) et arrière (14).

12. Dispositif selon l'une quelconque des revendications 9 à 11, **caractérisé en ce que** l'organe (12) d'accrochage comprend un mécanisme d'attache à déformation élastique faisant saillie sur la surface extérieure du boîtier (2).

13. Dispositif selon l'une quelconque des revendications 9 à 12, **caractérisé en ce que** l'organe (12) d'accrochage comprend un mécanisme d'attache à déformation élastique logé dans une cavité (13) du boîtier destinée à accueillir au moins partiellement le raccord (11) de sorte que le raccord (11) est au moins partiellement logé dans le boîtier sans faire saillie sur la surface extérieure du boîtier (2).

## Patentansprüche

1. Vakuumregelvorrichtung, welche ein Gehäuse (2) umfasst, in dem ein Fluidreislauf (3) angeordnet ist, der sich zwischen einem Eingang (4) und einem Ausgang (5) erstreckt, wobei der Kreislauf (3) ein Organ (6) zur Regelung des Vakuumniveaus umfasst, das durch ein am Gehäuse (2) beweglich angebrachtes Auswahlorgan (7) betätigbar ist, wobei die Vorrichtung außerdem einen Schalter (8, 15) zum Steuern des Einschaltens oder Ausschaltens der Vorrichtung über ein im Kreislauf (3) angeordnetes Absperrventil (15) umfasst, wobei der Schalter einen manuell betätigbaren Druckknopf (8) umfasst, wobei der Druckknopf (8) am Körper (2) in zwei entgegengesetzten Richtungen beweglich zwischen zwei Positionen, die der Einschalt- bzw. der Ausschaltposition entsprechen, angebracht ist, **dadurch gekennzeichnet, dass** ein erstes Ende (18) des Druckknopfes (8) in der Ausschaltposition in Bezug auf das Gehäuse (2) vorsteht, während ein zweites Ende (28) des Druckknopfes (8) in der Einschaltposition in Bezug auf das Gehäuse (2) vorsteht, wobei die Vorrichtung außerdem eine Sichtanzeige (9) der Einschalt- oder Ausschaltposition des Druckknopfes (8) umfasst, und dadurch, dass die Sichtanzeige (9) der Position einen Informationsträger (19) umfasst, der vom ersten (18) und zweiten Ende (28) des Druckknopfes (8) verschieden ist, wobei der Informationsträger (9) durch die Verlagerung mit dem Druckknopf (8) betätigt wird und je nachdem, ob sich der Druckknopf (8) in der Einschalt- oder in der Ausschaltposition befindet, eine erste Information (M) oder eine zweite Information (A) hervorhebt.

2. Vorrichtung nach Anspruch 1, **dadurch gekennzeichnet, dass** der Informationsträger (9) verschiebefest mit dem Druckknopf (8) verbunden ist.

3. Vorrichtung nach Anspruch 1 oder 2, **dadurch gekennzeichnet, dass** das Gehäuse (2) ein Fenster (16) aufweist, das sich auf dem Weg des Informationsträgers (9) bei dessen Verlagerungen mit dem Druckknopf (8) befindet, und dadurch, dass je nachdem, ob sich der Druckknopf (8) in der Einschalt- oder in der Ausschaltposition befindet, nur die erste Information (M) oder nur die zweite Information (A) des Informationsträgers (9) mit dem Fenster übereinstimmt.

4. Vorrichtung nach einem der Ansprüche 1 bis 3, **dadurch gekennzeichnet, dass** der Druckknopf (8) durch das Gehäuse (2) hindurch entlang einer Translationsachse (X) translatorisch beweglich ist, wobei die zwei Enden des Druckknopfes (8) jeweils auf einer von zwei einander gegenüberliegenden Seitenflächen des Gehäuses (2) münden, wobei das erste Ende (18) des Druckknopfes (8) in Bezug auf das Gehäuse (2) vorsteht, während das zweite Ende (28) des Druckknopfes (8) in Bezug auf das Gehäuse (2) zurückspringt oder mit ihm bündig ist, und umgekehrt.

5. Vorrichtung nach einem der Ansprüche 1 bis 4, **dadurch gekennzeichnet, dass** der Informationsträger (19) eine Platte umfasst, die starr mit dem Rest des Druckknopfes (8) verbunden ist.

6. Vorrichtung nach den Ansprüchen 4 und 5 in Kombination, **dadurch gekennzeichnet, dass** der Informationsträger (19) in Bezug auf das erste (18) und zweite (28) Ende des Druckknopfes (8) in einer Richtung quer zur Translationsachse (X) versetzt ist.

7. Vorrichtung nach einem der Ansprüche 1 bis 6, **dadurch gekennzeichnet, dass** der Informationsträger (9) die erste Information (M) oder die zweite Information (A) auf einer Seite des Gehäuses (2), Vorderseite genannt, hervorhebt, die das Auswahlorgan (7) aufweist.

8. Vorrichtung nach einem der Ansprüche 1 bis 7, **dadurch gekennzeichnet, dass** die erste Information (M) und die zweite Information (A) verschieden sind und jeweils wenigstens eines von wenigstens einer Farbe, einem alphanumerischen Zeichen und einem Text umfassen.

9. Vorrichtung nach einem der Ansprüche 1 bis 8, **dadurch gekennzeichnet, dass** der Eingang (4) und/oder der Ausgang (5) des Kreislaufs (3) ein Anschlussstück (11) umfasst, das demontierbar in Bezug auf das Gehäuse (2) angebracht ist, wobei das Gehäuse ein Organ (12, 13) zur lösbaren Befestigung eines Anschlussstücks (11) umfasst, um dieses Letztere zu halten, wenn es nicht am Eingang (4) oder am Ausgang (5) angebracht ist.

10. Vorrichtung nach Anspruch 9, **dadurch gekennzeichnet, dass** das Auswahlorgan (7) sich auf einer Seite (13) des Gehäuses (2) befindet, die Vorderseite genannt wird, wobei das Gehäuse (2) eine Hinterseite (14) aufweist, die der Vorderseite (13) gegenüberliegt, und dadurch, dass sich das Organ (12, 13) zur Befestigung des Anschlussstücks auf einer Seite des Gehäuses (2) befindet, die von der Hinterseite (14) verschieden ist.

11. Vorrichtung nach Anspruch 10, **dadurch gekennzeichnet, dass** sich das Organ (12, 13) zur Befestigung auf einer lateralen Seite des Gehäuses (2) befindet, die sich zwischen der Vorderseite (13) und der Hinterseite (14) befindet.

12. Vorrichtung nach einem der Ansprüche 9 bis 11, **dadurch gekennzeichnet, dass** das Organ (12) zur Befestigung einen Mechanismus zur Anbringung mit elastischer Verformung umfasst, der auf der Außenseite des Gehäuses (2) vorsteht.

13. Vorrichtung nach einem der Ansprüche 9 bis 12, **dadurch gekennzeichnet, dass** das Organ (12) zur Befestigung einen Mechanismus zur Anbringung mit elastischer Verformung umfasst, der in einer Vertiefung (13) des Gehäuses gelagert ist, die dazu bestimmt ist, das Anschlussstück (11) wenigstens teilweise aufzunehmen, so dass das Anschlussstück (11) wenigstens teilweise in dem Gehäuse gelagert ist, ohne auf der Außenseite des Gehäuses (2) vorzustehen.

## Claims

1. Vacuum regulator device comprising a housing (2) housing a fluid circuit (3) extending between an input (4) and an output (5), the circuit (3) comprising a member (6) for setting the vacuum level that can be actuated by a selection member (7) movably mounted on the housing (2), the device further comprising a switch (8, 15) for controlling the on state or the off state of the device via an isolation valve (15) situated on the circuit (3), the switch comprising a pushbutton (8) that can be actuated manually, the pushbutton (8) being mounted so as to be able to move on the body (2) in two opposite directions between two positions corresponding respectively to the on and off positions, **characterized in that** a first end (18) of the pushbutton (8) protrudes from the housing (2) in the off position whereas a second end (28) of the pushbutton (8) protrudes from the housing (2) in the on position, the device further comprising a visual indicator (9) of the on or off position of the pushbutton (8) and **in that** the visual position indicator (9) comprises an information support (19) distinct from the first (18) and second (28) ends of the pushbutton (8), the information support (9) being actuated by the displacement with the pushbutton (8) and respectively revealing a first information (M) or a second information (A) depending on whether the pushbutton (8) is in the on position or in the off position.

2. Device according to Claim 1, **characterized in that** the information support (9) is secured in displacement with a pushbutton (8).

3. Device according to Claim 1 or 2, **characterized in that** the housing (2) comprises a window (16) situated on the path of the information support (9) in its displacement with the pushbutton (8) and **in that** only the first information (M) or the second information (A) from the information support (9) coincides with the window depending on whether, respectively, the pushbutton (8) is in the on position or in the off position.

4. Device according to any one of Claims 1 to 3, **characterized in that** the pushbutton (8) is able to move in translation through the housing (2) along a translation axis (X), the two ends of the pushbutton (8) emerging respectively on two opposing lateral faces of the housing (2), the first end (18) of the pushbutton (8) protruding from the housing (2) when the second end (28) of the pushbutton (8) is set back from or flush with the housing (2) and vice versa.

5. Device according to any one of Claims 1 to 4, **characterized in that** the information support (19) comprises a panel rigidly linked to the rest of the pushbutton (8).

6. Device according to Claims 4 and 5 taken in combination, **characterized in that** the information support (19) is offset with respect to the first (18) and second (28) ends of the pushbutton (8) in a direction transverse to the axis (X) of translation.

7. Device according to any one of Claims 1 to 6, **characterized in that** the information support (9) reveals the first information (M) or the second information (A) on a face called front face of the housing (2) comprising the selection member (7).

8. Device according to any one of Claims 1 to 7, **characterized in that** the first information (M) and the second information (A) are distinct and each comprise at least one out of at least a colour, an alphanumeric symbol, and a text.

9. Device according to any one of Claims 1 to 8, **characterized in that** the input (4) and/or the output (5) of the circuit (3) comprises a coupling (11) mounted removably with respect to the housing (2), the housing comprising a member (12, 13) for removably attaching a coupling (11) to hold the latter when it is not mounted at the input (4) or the output (5).

10. Device according to Claim 9, **characterized in that** the selection member (7) is situated on a so-called front face (13) of the housing (2), the housing (2) comprising a rear face (14) opposite the front face (13) and **in that** said coupling attachment member (12, 13) is situated on a face of the housing (2) distinct from the rear face (14) .

11. Device according to Claim 10, **characterized in that** the attachment member (12, 13) is situated on a lateral face of the housing (2) situated between the front (13) and rear (14) faces.

12. Device according to any one of Claims 9 to 11, **characterized in that** the attachment member (12) comprises an elastically deformable attachment mechanism protruding on the outer surface of the housing (2).

13. Device according to any one of Claims 9 to 12, **characterized in that** the attachment member (12) comprises an elastically deformable attachment mechanism housed in a cavity (13) of the housing intended to at least partially accommodate the coupling (11) such that the coupling (11) is at least partially housed in the housing without protruding on the outer surface of the housing (2).
